# EUROPEAN PATENT SPECIFICATION

(11) **EP 4 061 454 B1**
(45) Date of publication and mention of the grant of the patent: **13.03.2024**
(21) Application number: 20808109.1
(22) Date of filing: 17.11.2020
(51) Int. Cl.: A61M 15/00

(54) **DRY POWDER INHALER WITH AN ADHERENCE MONITOR**
TROCKENPULVERINHALATOR MIT EINEM EINHALTUNGSMONITOR
INHALATEUR DE POUDRE SÈCHE COMPORTANT UN DISPOSITIF DE SURVEILLANCE D'ADHÉRENCE

(30) Priority: 18.11.2019 EP 19209858; 18.11.2019 EP 19209856; 18.11.2019 EP 19209857
(43) Date of publication of application: 28.09.2022
(73) Proprietor: Vectura Delivery Devices Limited, Chippenham, Wiltshire SN14 6FH (GB)
(72) Inventor: COTTON, Darryl, Cambridge, Cambridgeshire CB4 0GZ (GB); DEAMER, John, Cambridge, Cambridgeshire CB4 0GZ (GB); CLARKE, Roger, Cambridge, Cambridgeshire CB4 0GZ (GB); MELINIOTIS, Andreas, Cambridge, Cambridgeshire CB4 0GZ (GB); SMITH, Philip, Cambridge, Cambridgeshire CB4 0GZ (GB); SWANBURY, Philip, Cambridge, Cambridgeshire CB4 0GZ (GB); THOMAS, Seth, Cambridge, Cambridgeshire CB4 0GZ (GB)
(74) Representative: Clarke, Christopher John
(86) International application number: PCT/EP2020/082432
(87) International publication number: WO 2021/099329

(56) References cited:
- EP-A1- 3 552 647
- WO-A1-2016/156093
- US-A1- 2007 240 712
- US-A1- 2010 031 956
- US-A1- 2012 145 586
- US-A1- 2016 256 639
- US-A1- 2017 325 734
- US-A1- 2019 224 426

## Description

### Technical Field of the Invention

The present invention relates to an inhaler for dry powders containing an active substance for inhalation. In particular, the invention relates to an inhaler for use with a module for monitoring a patient's use of the inhaler.

### Background to the Invention

Dry powder inhalers (DPIs) provide an attractive method for administering medicaments, for example to treat local diseases of the airway or to deliver drugs to the bloodstream via the lungs. The medicament is commonly provided as individual doses, such as a strip having a plurality of blisters, for example as disclosed in WO13/175177.

The efficacy of treatment is dependent on the patient using the inhaler correctly and as prescribed. Consequently, there is increasing interest in monitoring patient adherence, i.e. whether the patient takes the prescribed number of doses per day, e.g. once or twice daily.

DPIs typically have a dose counter, either in the form of numbers printed onto the blister strip or as a separate mechanism which counts up or down each time the inhaler is actuated. For example, US2010/031956 discloses an inhaler with numbers or symbols that are marked directly on the blister strip. However, while this tells the patient the number of remaining doses, so that they know when a new inhaler is required, it does not help the caregiver to monitor adherence, because the dose number is only seen by the patient.

Therefore, devices have been developed that provide adherence information. The monitor typically determines when the inhaler has been actuated (e.g. optical or by electric contacts) and hence counts the number of doses that have been taken. However, if an actuation is not counted correctly (for example, an aborted actuation is counted, or an actuation is not counted due to sensor error) then monitor could display the incorrect dose number.

Since adherence monitors typically contain expensive sensors, electronics etc., they are often provided as separate add-on modules which couple to the inhaler. DPIs typically contain a month's supply of medication. Thus, when the medication in the inhaler has been used up, the monitor can be detached and then re-attached to a new inhaler. For example, US2017/325734 and US2010/0192948 disclose detachable adherence monitors for DPIs which record when the inhaler is used, by mechanically or optically detecting movement of its mechanical parts. However, the fact the monitor is detachable has the disadvantage that the patient could remove it before all of the doses in the inhaler are used up. Thus, if the monitor is later re-attached to the same inhaler, it cannot tell how many doses have been dispensed during the period in which it was detached. US2007/240712 discloses an inhalation device for delivering a therapeutic agent, e.g. insulin, to e.g. a diabetic patient, which has at least two vibrators for selectively coupling with two or more dose packs containing the same or different dose quantities of the therapeutic agent to make up a desired dose of the therapeutic agent.

### Brief description of the invention

The present invention seeks to overcome these drawbacks. The invention is defined by the appended claims.

The invention provides a dry powder inhaler and a monitor, the inhaler having a mouthpiece and a housing which contains a blister pack comprising a plurality of blisters, wherein a blister, or group of blisters, provides a dose of powdered medicament for inhalation, wherein the blister pack has non-numerical indicia which encode an individual, unique number that is associated with each dose. The inhaler is adapted for removably mounting the monitor. The monitor comprises one or more sensors, preferably optical sensors, which are configured to read the non-numerical indicia, and a controller and memory which are configured to process and/or store information read by the sensors, so that the monitor can determine the unique number of each dose. The inhaler may have a mouthpiece cover. The blister pack may be a blister strip.

The invention also provides a monitor which is removably attachable to an inhaler of the invention, wherein the monitor comprises one or more sensors, preferably optical sensors, which are configured to read the non-numerical indicia, and a controller and memory which are configured to process and/or store information read by the sensors, so that the monitor can determine the unique number of each dose.

The monitor reads the indicium on the blister pack associated with the current dose. From this it determines the unique number of that dose, and consequently the number of doses that have been dispensed or remain to be dispensed. The dose number is unique within the blister pack (e.g. one of the numbers from 1 to 30). Since the monitor determines the dose number by reading the unique indicium of the dose from the blister pack, rather than by counting actuations of the inhaler, there is no possibility of the dose number being incorrect. In other words, the monitor determines the dose number in an absolute manner by reading the dose number from the blister pack, rather than in a relative manner, such as by counting uses of the inhaler as in US2010/0192948.

The monitor may clip onto the housing of the inhaler. The monitor may be supplied separately from the inhaler, so that a single monitor may be used with many different inhalers. The patient could remove the monitor and later re-attach it; or the battery in the monitor could run out and need to be recharged. Since the monitor reads the unique dose number, it will still record the correct dose number, regardless of whether the inhaler was used while the monitor was detached or while the battery was flat. Alternatively, the monitor may be intended for use with a single inhaler only, in which case it may be permanently attached to the inhaler, e.g. by ultrasonic welding or glueing. Again, if the battery runs out, the dose counter will still record the correct dose number once it has been re-charged, even if the inhaler was used while the battery was flat.

The inhaler may have a mechanism for advancing the blister pack and for opening the blisters which is operated by an actuator. The opening mechanism is suitably a piercer which is mounted on the underside of the mouthpiece. The actuator drives the indexing mechanism to move one or more blisters into alignment with the piercer and which then moves the mouthpiece relative to the housing so that the piercer pierces the aligned blister(s). The actuator may be a lever which causes indexing of the blister pack and piercing of the blisters. Alternatively, the actuator may be formed as part of, or be connected to, the mouthpiece cover, so that rotation of the cover causes indexing of the blister pack and piercing of the blisters.

For example, the inhaler may be of the type described in WO13/175177 which has a blister strip that is advanced by pivoting the mouthpiece cover. In a first stage, moving the cover from the closed position to an intermediate position causes the blister strip to be advanced; and in a second stage, moving the cover from the intermediate position to the open position operates the piercer.

The inhaler may be configured to index and pierce one blister on each actuation. Alternatively, it may index and pierce two (or more) blisters on each actuation, and thereby delivertwo (or more) different formulations simultaneously, or a double (or multiple) amount of a single formulation. Thus a dose of medicament may be provided by a single blister, in which case an individual non-numerical indicium is associated with each blister. Alternatively, a dose may be provided by two (or more) blisters, in which case an individual non-numerical indicium is associated with each pair (or group) of blisters.

The indicia may be accessible to be read though an aperture and / or transparent section in the housing of the inhaler. The sensors for reading the indicia may comprise one or more optical sensors. The monitor interprets the output from the sensors.

The indicia may be in the form of blocks of ink printed onto the blister strip and gaps between the block, for example a bar code or 2D matrix code. The monitor may read an indicium by simply capturing an image of it. Alternatively, the monitor may be configured to scan the indicia as the blister pack is indexed. The sensor may be an optical sensor which scans the indicia as the blister strip is indexed past it. The sensor may detect transitions from light to dark and / or vice versa as the blister strip is moved past the sensor.

The indicia may additionally provide information which allows the direction of motion (i.e. forwards or reverse) and / or the position of the blister pack (e.g. within an indexing step) to be determined by the monitor.

In one embodiment, the indicia are made up of three rows of blocks printed onto a blister strip, and the monitor has three corresponding optical sensors for reading the three rows of printed blocks. Two rows provide information which allows the direction of motion (i.e. forwards or reverse) and the position of the blister strip (e.g. within an indexing step) to be determined, and the third row provides the unique number associated with each dose. The sensors may be photomicrosensors which each emit light and detect the light reflected from the indicia on the blister strip. Alternatively, the monitor may have a single light source, such as an LED, and three photodetectors; a single LED has lower power consumption.

The inside of the mouthpiece cover may have markings for indicating its position and / or direction of motion, for example depressions or cavities formed by moulding. The monitor may have one or more further sensors, for example optical sensor(s), on its outer side that are configured to read the markings on the inside of the cover. The monitor can thereby determine whether the cover has been fully opened, and hence, for example, whether the blister was pierced, or whether the operation was aborted before piercing.

In another embodiment, the indicia are matrix codes made up of two rows of blocks printed onto a blister strip, and the monitor has two corresponding optical sensors for reading the two rows of printed blocks. Alternatively, the indicia are matrix codes made up of three rows of blocks printed onto a blister strip, and the monitor has three corresponding optical sensors for reading the three rows of printed blocks. The matrix codes provide the unique number associated with each dose. The monitor has one or more switches on its outer side, and the inside of the mouthpiece cover has one or more cams which actuate the switch(es) on the monitor as the cover is moved. The switches allow the monitor to determine the position and / or direction of motion of the cover.

The monitor may have a sensor, such as a pressure sensor, which is connected to the mouthpiece, either via a passage in the inhaler or via a separate external tube, so that the monitor can additionally detect whether the user has inhaled on the mouthpiece.

### Brief Description of the Figures

The invention will now be further described with reference to the Figures, wherein:
Figure 1A shows an inhaler and a monitor according to the invention, with the cover in the closed position, so that the mouthpiece is covered.
Figure 1B shows the inhaler of Figure 1A with the cover in the open position so that the mouthpiece is exposed.
Figure 1C shows another view of the inhaler of Figure 1A, from a different angle.
Figure 1D shows the inhaler of Figure 1A with the monitor removed and the cover closed.
Figures 2A and 2B show the monitor removed from the inhaler.
Figure 3 shows a blister strip with a 1D bar code.
Figure 4 is a cross-section through the inhaler of Figure 1 which shows the locations of the code and number corresponding to the current blister that is in use.
Figure 5 shows a blister strip with an alternative type of indicia.
Figure 6 shows a blister strip with a 2D matrix code.
Figure 7 shows part of a blister strip which has a bar code aligned along its length.
Figure 8 shows a preferred embodiment of a printed indicium.
Figure 9 shows the sensor output voltages from each of the three photomicrosensors corresponding to the indicium of Figure 8.
Figure 10A shows markings in the form of cavities on the inside of the mouthpiece cover.
Figure 10B shows the corresponding sensor output voltages as a function of the opening angle of the cover.
Figure 11 shows part of a blister strip which has indicia in the form of 2D matrix codes.
Figure 12A shows an embodiment of the monitor that has switches on its outer side.
Figure 12B shows an embodiment of the mouthpiece cover that has cams on its inside that actuate the switches.
Figure 13 shows further preferred embodiments of printed indicia in the form of 2D matrix codes.
Figure 14 shows a series of displays on a mobile phone with an app that receives data from the monitor during use of the inhaler.

### Detailed Description of the Invention

In the context of dry powder inhalers, the term "adherence" is normally used to refer to whether the patient takes the prescribed number of doses per day, e.g. once or twice daily.

The term "compliance" is normally used to refer to whether the patient uses their inhaler correctly, e.g. if they inhale sufficiently strongly to entrain the powder and disperse it into particles that reach the lung. Consequently, a monitor may be designed to measure adherence and / or compliance, according to the type of sensors that it uses, and how they are configured. In the present application, the term "monitor" therefore refers to a module having one or more sensors that is designed to measure and capture information relating to adherence, and additionally may measure and capture compliance information. The monitor does not perform any of the functions associated with dosing the medication, such as a piercing or opening blisters / capsules, de-agglomerating the powder or providing a breath-actuation mechanism. The inhaler therefore operates to dispense powder whether the monitor is present or not.

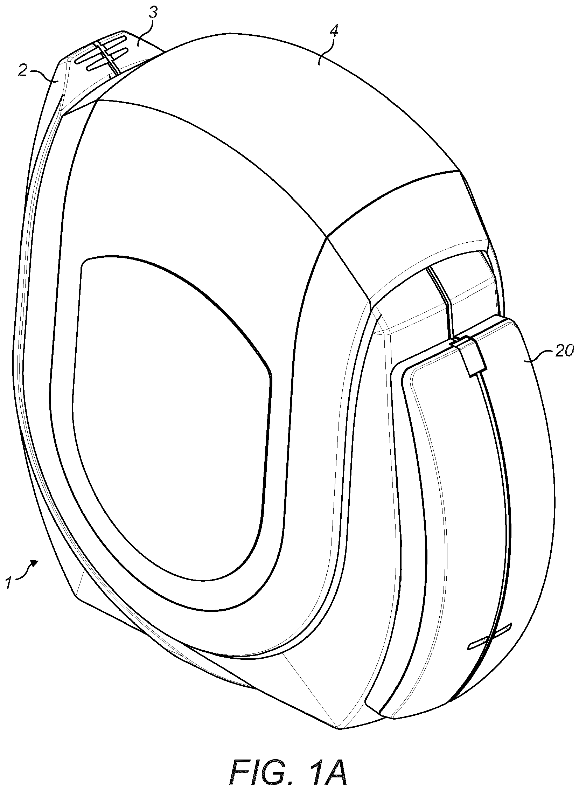

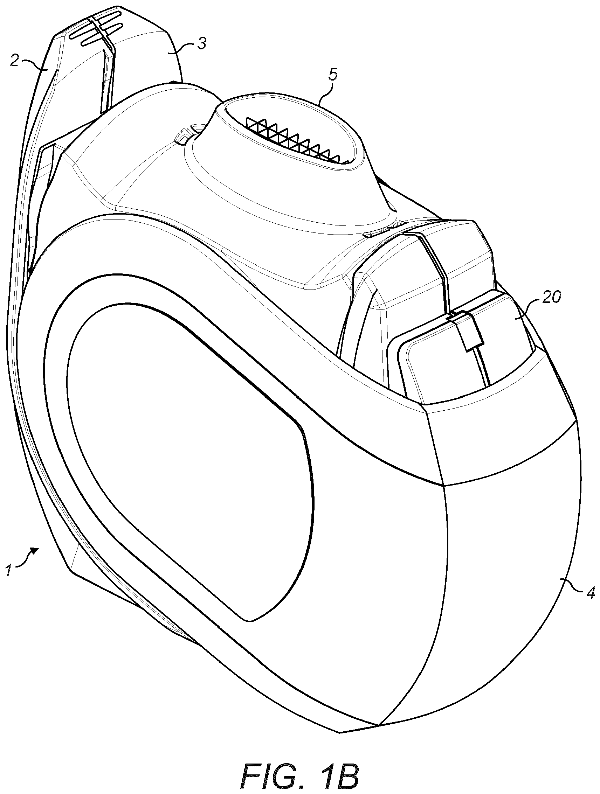

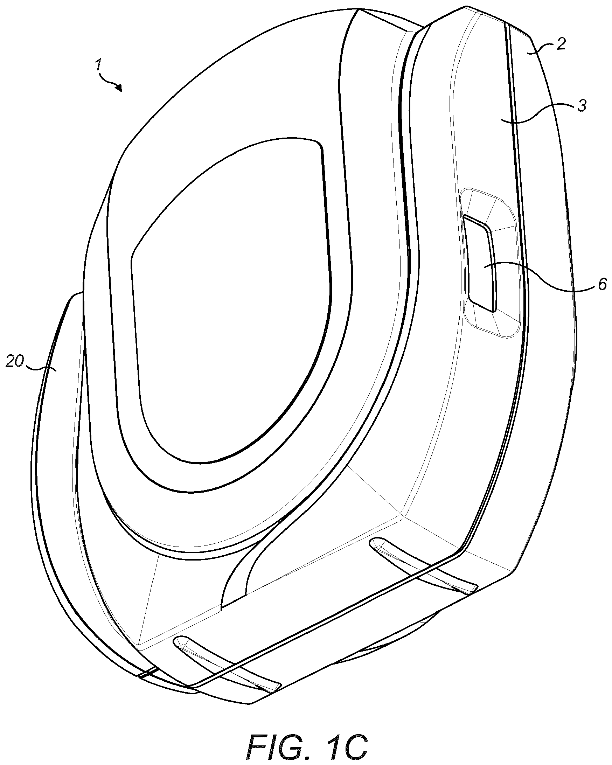

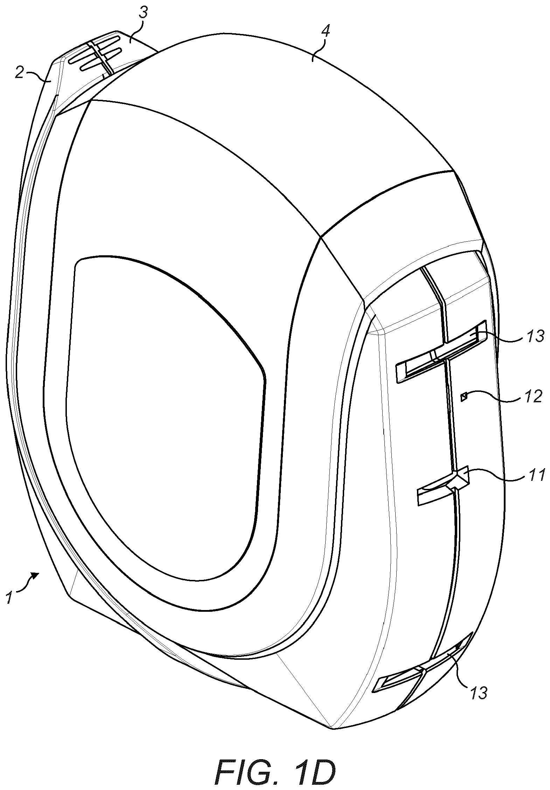

An inhaler and monitor according to the invention are shown in Figure 1. Figures 1A and 1C show an inhaler with a monitor attached from two different angles, with the mouthpiece cover in the closed position. Figure 1B shows the inhaler with the mouthpiece cover in the open position so that the mouthpiece is visible. Figure 1D shows the inhaler with the monitor removed and the cover closed.

The inhaler shown in Figure 1 is an "open-inhale-close" inhaler of the type described in WO13/175177. However, the invention is not limited to this type of inhaler, and for example, could equally be used with an inhaler which has a passive mouthpiece cover, and a separate actuating lever, as described for example in WO13/175176; or with an inhaler which has a blister disk instead of a blister strip.

The inhaler **1** shown in Figure 1A is constructed from two shell parts **2, 3** which are joined together to form a housing that contains a blister strip. A detachable monitor **20** is attached to one side of the inhaler. A mouthpiece cover **4** is mounted onto the housing. The cover **4** can be rotated (through an angle somewhat greater than 90°) from the closed position in which it covers and protects a mouthpiece **5** (Figure 1A) to a fully open position, in which the mouthpiece is exposed so that the user can inhale a dose of medicament (Figure 1B).

The inhaler has a strip of blisters containing powdered medicament for inhalation. The blister strip is typically cold formed from a ductile foil laminate or a plastics material and includes a pierceable lid, typically foil or a foil laminate (e.g. aluminium), which is heat-sealed around the periphery of the blister after the dose of medicament has been introduced during manufacture. The mouthpiece is formed as part of a component which is pivotally mounted to the housing. A piercer is located directly underneath the mouthpiece. The cover is selectively coupled to a blister strip indexing mechanism and to the mouthpiece component. Moving the cover from the closed position to an intermediate position (the first stage of opening) causes the indexing mechanism to advance the blister strip. Then, once an unused blister is in position beneath the piercer, the indexing mechanism is disengaged. The first stage is reversible, i.e. the user can abort the actuation of the inhaler (since piercing has not yet occurred) simply by closing the cover, which moves the blister strip back to its previous position. Moving the cover from the intermediate position to the fully open position (the second stage) causes the mouthpiece component to pivot downwards so that the piercer pierces the aligned blister. The user then inhales through the mouthpiece, which aerosolizes the powder in the pierced blister.

The inhaler may be configured to index and pierce one blister on each actuation. Alternatively, it may index and pierce two (or more) blisters on each actuation, and thereby deliver two (or more) different formulations or medicaments simultaneously. Alternatively, it may for example deliver a large amount of the medicament, e.g. double or triple, by piercing two or three blisters. In other words, a dose of medicament may be provided by one blister, or by more than one blister, for example two blisters with different medicaments which are delivered in a single actuation.

Figure 1C shows a view of the inhaler of Figure 1A, from a different angle. The housing has an opening 6 on the opposite side from the monitor 20. This allows the user to see a number printed on the blister strip. When the user actuates the inhaler by opening the cover, the indexing mechanism advances the blister strip by one dose. As a result, the number on the blister strip that is visible to the user sequentially decreases (or increases). The numbers therefore provide a dose counter which displays the number of doses remaining (or that remain to be dispensed) in the inhaler.

Figure 1D shows the inhaler with the monitor having been removed. An aperture 11 is visible in the wall of the housing where the monitor was attached. The aperture allows the monitor to read the indicia on the blister strip. The aperture may have a transparent window or it may simply be open. The aperture is spaced apart from the dispensing position, in the 'upstream' direction (i.e. before the blister is opened), so that the indicia that are read by the monitor are on the unused part of the blister strip. However, the aperture could be located at or near the dispensing position, or 'downstream' (i.e. after the blister has been opened) of the dispensing position, provided that the blister strip is arranged to take this into account, as is explained below. This is less preferred, since at least in theory, there could be traces of powder on the used part of the blister strip, in particular on the indicia, which could lead to errors in reading them. Instead of having an aperture, a portion of the housing may be transparent such that the indicia are visible externally.

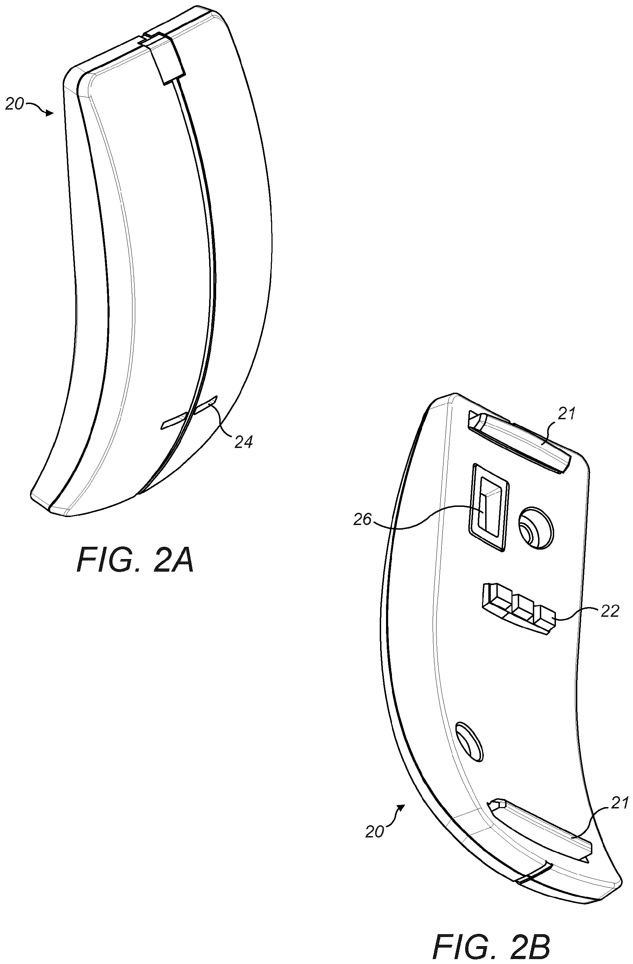

There is also a small orifice **12** in the wall of the housing, which allows a pressure sensor **26** (shown in Figure 2B) in the monitor to connect to the mouthpiece via a channel inside the inhaler. The housing also has two slots **13** for mounting the monitor as described below.

Figure 2 shows the monitor on its own (i.e. detached from the inhaler). Figure 2A shows the outer side of the monitor, and Figure 2B shows the inside face of the monitor (i.e. the side which abuts the inhaler when the monitor is attached). The monitor **20** has two clips **21** which fit into the corresponding slots **13** in the housing, and thereby hold the monitor in place when attached to the inhaler. The clips and slots allow the monitor to be detachably mounted on the inhaler, e.g. by an interference fit. A detachable monitor has the advantage that it may be supplied separately from the inhaler, and that a single monitor may be used with many different inhalers. Thus, when the medication in the inhaler has been used up, the monitor can be detached and then re-attached to a new inhaler. The new inhaler could be identical to the used one. Alternatively, it could contain a different dose strength or a different active, or even be a different type of inhaler, provided that it is compatible with the monitor.

The monitor has one or more sensors **22**, such as optical sensors, for reading the indicia on the blister strip. In the embodiment shown in Figure 2, the monitor has three photomicrosensors, each consisting of a light source, such as a light emitting diode (LED) and a photodetector with an attached lightguide. The lightguide channels light from the LED to the blister strip where it is reflected back through the lightguide to the photodetector. The monitor uses the reflected light signals to determine the unique identity of the particular blister in a manner that is described in detail below, so that the number of doses that have been dispensed or that remain to be dispensed can be read directly from the blister strip. This is advantageous over monitors which determine the number of doses by counting when the inhaler is actuated. For example, the patient could remove the monitor and later re-attach it; or the battery in the monitor could run out and need to be recharged. Since the monitor reads the unique dose number of the blister, it will still record the correct dose number, regardless of whether the inhaler was used while the monitor was detached or while the battery was flat. The monitor may also have further optical sensors **24** whose purpose is explained below.

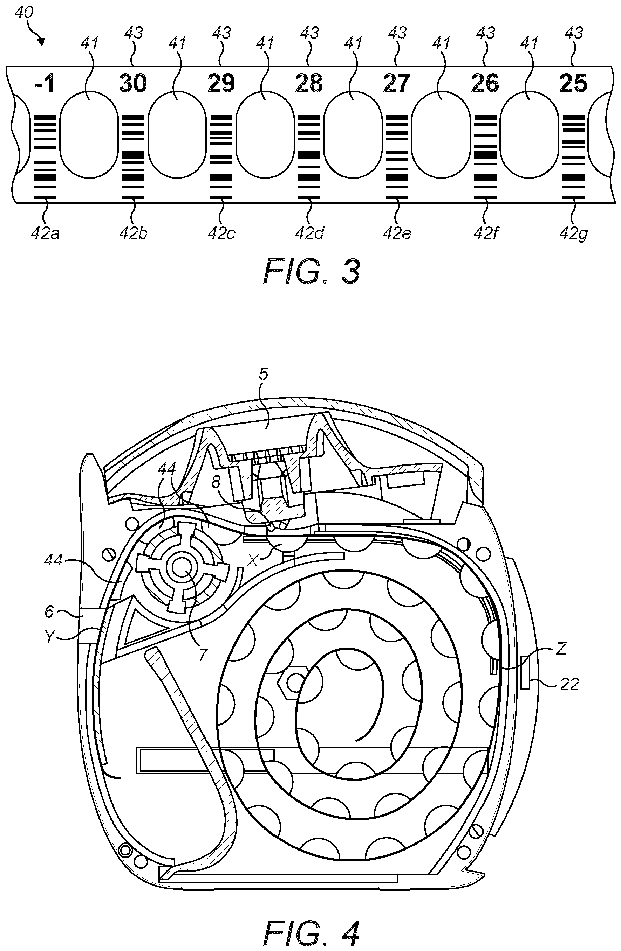

Figure 3 shows a blister strip **40** having a plurality of separate blisters **41** containing powdered medicament. A series of 1D bar codes **42a, 42b, 42c, 42d, 42e, 42f, 42g** is printed onto the blister strip. The width and spacing of the lines in the bar codes define an individual number associated with each blister. The blister strip additionally has printed numbers **43.** Each individual blister is associated with one of the numbers and one of the bar codes.

However, neither the number nor the bar code is located adjacent to the blister with which it is associated. The reason for this is apparent from Figure 4, which shows a cross-section through the inhaler of Figure 1. The blister strip has a leading end without any blisters, and then three empty blisters **44** before the first blister **X** filled with medicament. This is because the indexing mechanism advances the blister strip by means of a drive wheel **7** which is located in advance (downstream) of the piercer **8.**

In the situation shown in Figure 4, the first dose is about to be taken and the first filled blister **X** is situated directly beneath the mouthpiece **5** and the piercer **8.** The number **Y** associated with this blister is 30, because this is the total number of doses in the inhaler before use. The empty blisters **44** upstream of this have negative numbers (-1 etc.), which may be used during production for control checks. However, the opening **6** through which the user reads the number of doses remaining is not located adjacent to the mouthpiece, but in a sidewall of the housing close to the drive wheel **7.** Consequently, the number **Y** associated with blister **X** is actually four blisters further along the blister strip in the downstream direction. Similarly, the sensor **22** which reads the bar code **Z** associated with blister **X** is located on the opposite sidewall of the housing. Thus the bar code **Z** is actually four blisters upstream of blister **X** (i.e. eight blisters upstream of the number **Y**). Bar codes associated with the empty blisters may also be used for control checks during production.

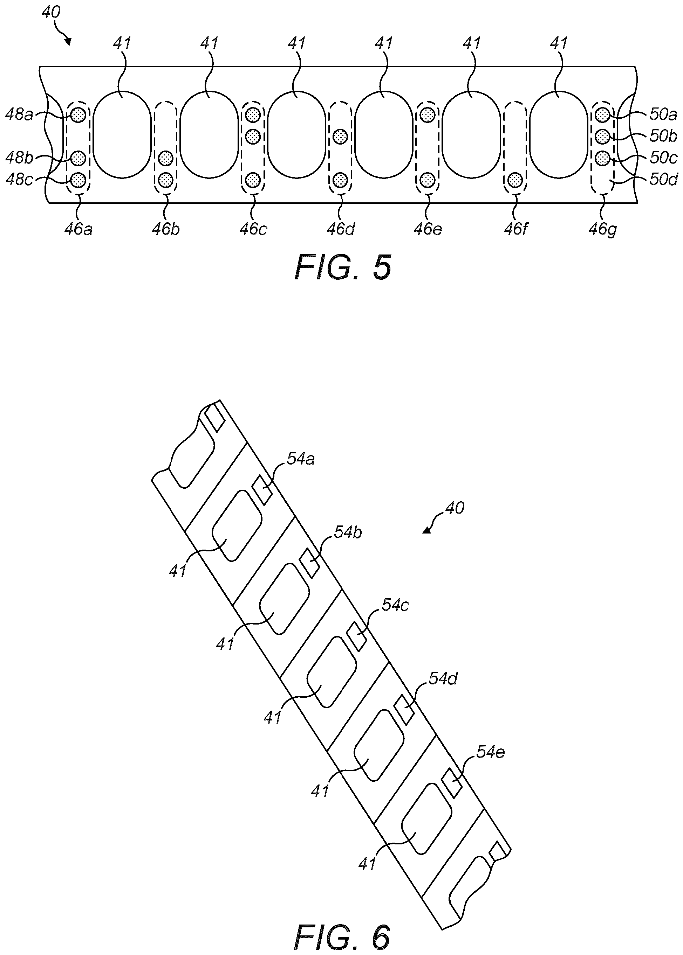

Figure 5 shows a further blister strip **40** which has a different type of indicia. Each indicium **46a, 46b, 46c, 46d, 46e, 46e, 46f, 46g** comprises a series of spaced apart dots **48a, 48b, 48c** which are located in four possible positions **50a, 50b, 50c, 50d.** The presence of a dot represents a 0 and the absence of a dot represents a 1 (or vice versa), so that each indicium represents an individual binary number associated with a blister **41,** in this case from 0 to 15. The indicia **46a, 46b, 46c, 46d, 46e, 46e, 46f, 46g** correspond to numbers 13 to 7 respectively. Five or six dots / spaces would be required for a blister strip having thirty or sixty blisters respectively (2⁵=32, 2⁶= 64).

Figure 6 shows a blister strip **40** with 2D matrix codes **54a, 54b, 54c, 54d, 54e,** for example a QR^{®} code. Again, the code defines an individual number associated with each blister **41.**

The indicia may be printed using an ink compatible with the blister strip. The monitor detects the difference between the dark (non-reflective) ink and the reflective strip. Alternatively, a different type of coating may be used instead of ink, for example, one that is magnetic or fluorescent, which is read by a corresponding sensor in the monitor. Another possibility is to form the indicia by creating bumps and / or dimples in the blister strip, or by laser ablation. Indeed, any suitable type of marking could be used, coupled with an appropriate sensing system, for example capacitive sensing, inductive sensing using a Hall effect sensor, a reflective photosensor (which could use visible or non-visible wavelengths), or a transmissive sensor. The monitor may read the indicium by simply capturing an image of it.

Although the indicia in Figures 3 and 5 extend across the blister strip, they could alternatively extend along the blister strip, i.e. rotated through 90 degrees. In this orientation, the sensor does not need to capture an image of each indicium but can instead scan it as moves past the sensor when the blister strip is indexed.

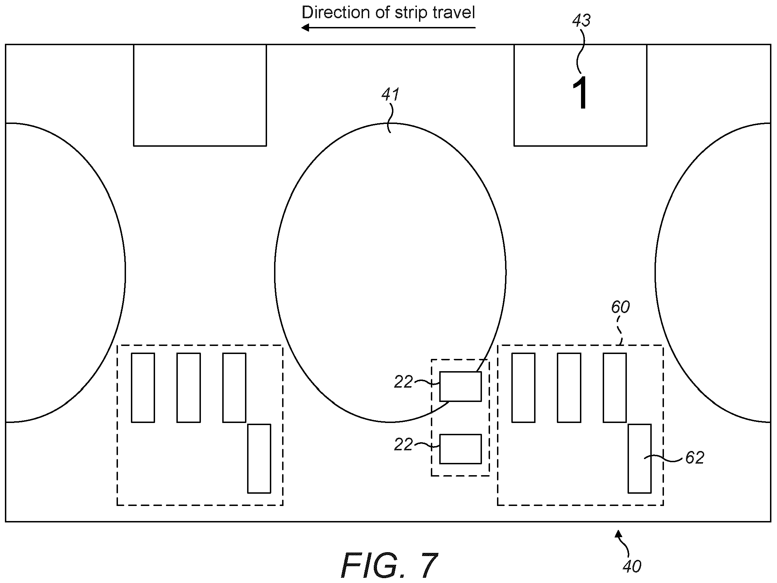

Figures 7 to 10 relate to a first embodiment in which the blister strip **40** has printed numbers **43** and non-numerical indicia in the form of bar codes **60** oriented along the length of the blister strip. Each indicium also has additional features **62** whose purpose is to indicate whether the bar code has been fully read and / or to allow the direction of motion of the blister strip to be determined, in a manner which is described below. Figure 7 also indicates the position of the sensors **22** relative to blister strip. In this case, there are two photomicrosensors, one to read the bar code **60** and the other to read the additional features **62.** The sensors **22** are held in a fixed position above the aperture **11** in the housing of the inhaler, constrained by the clips that connect the monitor to the housing. In Figure 7, the position of the sensors **22** relative to the indicia **60**, **62** corresponds to the situation before the blister strip is indexed. When the user actuates the inhaler, the blister strip **40** is indexed from right to left so that the indicia move past the sensors **22** which record the intensity of the reflected light received by the photodetector.

The user could abort actuation of the inhaler part way through the first stage of opening the cover and close it again, without having pierced a blister **41**, so that the motion of the blister strip is reversed. In this case, without the additional features **62**, the bar code might not be correctly read. The monitor could record an incorrect number if, for example, half of the code was scanned as the blister strip moves forwards, and then the same half scanned again in reverse as the blister strip moves backwards during an aborted actuation. By having additional features 60 which indicate the direction of motion of the blister strip and / or that the bar code has been fully read, the monitor can distinguish a normal actuation from an aborted actuation, and therefore can discount the latter.

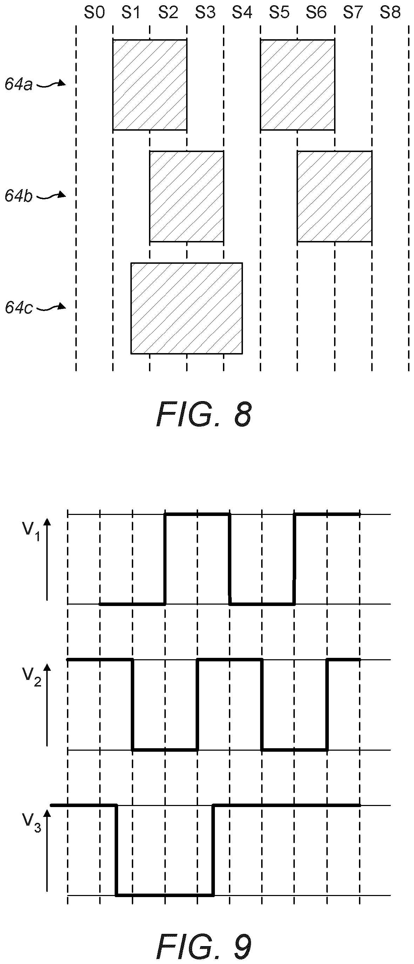

Figure 8 shows a preferred embodiment of an indicium which defines an individual number associated with each blister in a 30 dose blister strip. The indicium also has features which enable the monitor to determine the direction of motion of the blister strip and the extent to which the indicium has been read. The indicium consists of three rows of nine regions (labelled S0 - S8). In each row, the regions are either printed black to form rectangular blocks, or left blank so that the reflective metal foil is exposed. The monitor correspondingly has three sensors, one for each row. The first two rows **64a**, **64b** act as an encoder for determining the direction of motion of the blister strip, and also define the boundaries of the regions. The third row **64c** encodes a unique number associated with each individual blister; this number may also be printed on the blister strip, so that it can be read by the user through the opening **6** in the housing on the opposite side of the inhaler from the monitor, as with the blister strip of Figure 3.

The first two rows **64a, 64b** are identical for each blister. In the first row **64a**, regions S1, S2, S5 and S6 are printed, whereas S3, S4, S7 and S8 are blank. In the second row **64b,** regions S2, S3, S6 and S7 are printed, whereas S1, S4, S5 and S8 are blank. In each case, there is also blank space between the indicia on adjacent blisters, indicated by region S0 on the left side of the indicium in Figure 8. The third row **64c** encodes a five digit binary number using five blocks that are offset by half of the width of a region from the first and second rows.

Figure 9 shows the output voltage signals **V₁**, **V₂**, **V₃** respectively from each of the three sensors resulting from the three rows of blocks **64a, 64b, 64c** of Figure 8. The output voltage is proportional to the intensity of the light reflected from the indicium that is received by each sensor. The binary state is determined by comparing the output voltage to a threshold level. If the output voltage is higher than the threshold voltage then the state is 1 and if the voltage is lower the state is 0.

The output voltages **V₁**, **V₂**, **V₃** are input to the processor in the monitor, which identifies transitions from low to high or high to low voltage. A transition from high voltage (i.e. blank region of blister strip) to low voltage (i.e. a printed region) from the first sensor is assigned a value of 1, and a transition from low to high is assigned a value of 2. Similarly, high to low and low to high transitions on the second sensor are assigned values of 3 and 4 respectively.

The third row encodes a binary number. The printed blocks are offset by half a region compared to the first and second rows, so that voltage transitions occur within a region rather than at the start / end. The encoder (first and second) rows thereby define boundaries. Consequently, it is possible to distinguish between the transitions resulting from a single printed (or reflective) block and two consecutive printed (or reflective) blocks in the third row. The transitions which are detected within regions S1 to S5 in the third row are classified to form a five digit binary number. A region with a transition (either from high to low voltage or from low to high) represents a binary 1, and a region with no transition represents a binary 0. With a 30 dose blister strip (i.e. a five digit binary number), region S6 is redundant and is ignored by the monitor. However, if the blister strip contained sixty doses, a transition in region S6 would provide a sixth binary digit. The seventh region in the third row is always blank, in order to reset in preparation for the next blister in a defined position. Thus a transition in region S7 (which occurs if a six digit binary number ends with a 1) is always ignored.

The transitions and outputs resulting from the indicium of Figure 8 when the blister strip is indexed through a complete actuation are shown in Table 1 below. The third row encodes the binary number 10010, i.e. 18.

**Table 1**

| **Region** | S0 | S1 | | S2 | | S3 | | S4 | | S5 | | S6 | | S7 | | S8 | S9 |
|---|---|---|---|---|---|---|---|---|---|---|---|---|---|---|---|---|---|
| **First row** | | | | | | | | | | | | | | | | | |
| **First sensor voltage** | High | Low | | Low | | High | | High | | Low | | Low | | High | | High | High |
| **Transition type** | 1 | | | 2 | | | | 1 | | | | 2 | | | | | |
| **Second row** | | | | | | | | | | | | | | | | | |
| **Second sensor voltage** | High | High | | Low | | Low | | High | | High | | Low | | Low | | High | High |
| **Transition** | | 3 | | | | 4 | | | | 3 | | | | 4 | | | |
| **Third row** | | | | | | | | | | | | | | | | | |
| **Third sensor voltage** | High | | Low | | Low | | Low | | High | | High | | High | | High | | High |
| **Transition** | | Y | | N | | N | | Y | | N | | N | | N | | N | N |
| **Binary number** | | 1 | | 0 | | 0 | | 1 | | 0 | | - | | - | | - | |

One transition occurs at the beginning of each region, with the sequence 13241324. In the same manner, indexing the strip backwards results in the reverse sequence 42314231. An incomplete actuation results in only part of the sequence being recorded, for example 132413. If the actuation is then aborted and the strip indexed backwards, the sequence would be 423142. The monitor is able to distinguish forwards and backwards motion of the blister strip because 1 is always followed by 3 when indexing forwards, whereas 1 is followed by 4 when indexing backwards, or by 2 in the case that motion is reversed before a transition occurs in the second row.

If the blister strip is indexed in the reverse direction, the monitor deletes corresponding digit in the binary number that had been recorded, so that no false reading is taken. The user could reverse the direction of motion of the cover part way through actuation, but then go on to complete the full actuation. For, example, if actuation was reversed from S4 back to S3, and then continued forwards again, the indicium would be read as shown in Table 2. (In the Direction row, F and R represent forward and reverse motion respectively).

**Table 2**

| **Region** | S0 | S1 | S2 | S3 | S4 | S3 | S4 | S5 | | S6 | | S7 | | S8 | S9 |
|---|---|---|---|---|---|---|---|---|---|---|---|---|---|---|---|
| **First row** | | | | | | | | | | | | | | | |
| **Transition type** | 1 | | 2 | | | | 1 | | | 2 | | | | | |
| **Second row** | | | | | | | | | | | | | | | |
| **Transition** | | 3 | | 4 | | 3 | 4 | 3 | | | | 4 | | | |
| **Direction** | | F | | | F | R | F | F | | | | F | | | |
| **Third row** | | | | | | | | | | | | | | | |
| **Transition** | | Y | N | N | Y | Y | Y | N | | N | | N | | N | N |
| **Binary number** | | 1 | 0 | 0 | 1 | 1 | 1 | 0 | | - | | - | | - | |

The output from the first two rows is 1324341324. The sequence 434 indicates reverse motion for one region, followed by forward motion. The transitions in the third row would appear to give the binary output as 1001110, i.e. with two extra 1s. However, because the monitor knows that motion was reversed for one region, the binary digit that was recorded during the reverse motion and the digit immediately preceding it are deleted. Thus, the corrected binary output is 10010, as before. In effect, since the first and second sensors together always record one transition at the beginning of each of the regions S1 to S8, the monitor knows which part of the number code in the third row is being read, and hence can ignore the erroneous and repeated digits.

In summary, the first two rows provide the position and direction of motion of the blister strip; the third row, provides an encoded an individual number associated with each blister. Since the monitor knows the position and direction of motion of the blister strip, the encoded number is read correctly, even if actuation is aborted or reversed part way through. Moreover, using region boundaries defined by the first two rows when reading the third row means that any variation in the speed of actuation has no effect on how the number is read. This provides an advantage over a standard 1D barcode, where a constant indexing speed would be required in order to determine the bar widths.

Alternatively, the indicia could have a grey printed region to give a reflectance intermediate between those of the blank reflective and black printed parts. Two threshold voltages could be used to distinguish three states (0, 1 and 2). This has the advantage that a single row contains more information and so can be used to define the regions and identify the direction of motion. For example, the first row may consist of repeated blocks of blank, grey and black, i.e. 012 012, so that 0 followed by 1 indicates forward motion and 0 followed by 2 indicates reverse motion. Consequently only two rows are required, and correspondingly, two sensors in the monitor, instead of three. On the other hand using only black printing has the advantage of being more tolerant to the signal variations from the blister strip.

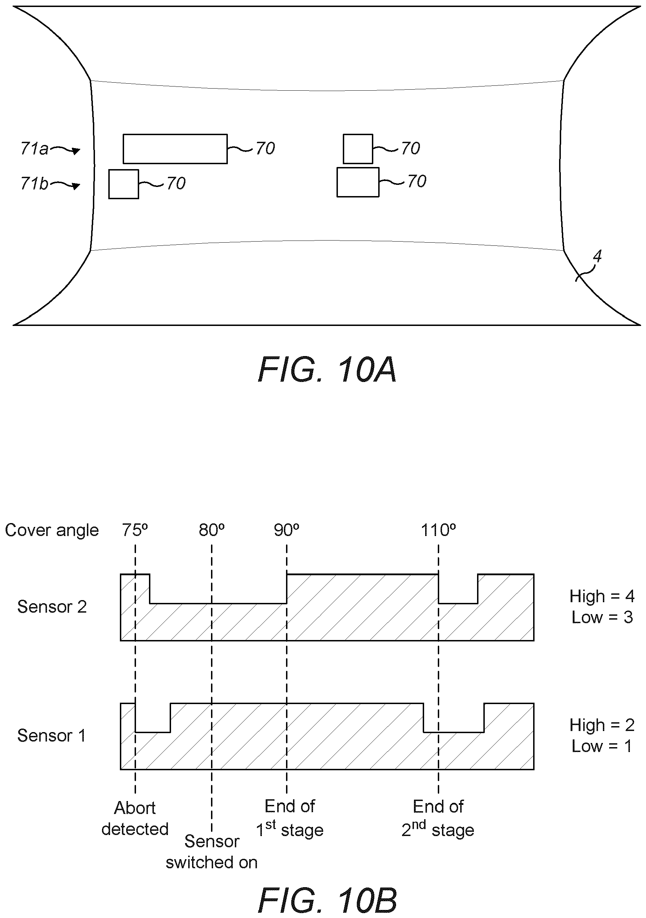

The monitor may also have an external optical sensor **24,** for example in a recess towards the lower edge of its outer side (see in Figure 2A). In this case, the inside of the mouthpiece cover **4** also has markings **70** as shown in Figure 10A, for example depressions or cavities that are formed by moulding. Alternatively, the markings could be printed or embossed. The external optical sensor detects changes in the intensity of the reflected light as the markings pass over it during the second stage of opening of the cover. A cavity is darker, so the sensor voltage output is low. In this way, the monitor can determine whether the cover has been fully opened so that the blister was pierced, or whether actuation was aborted before piercing. Two such sensors may be used, in combination with two sets of markings **71a, 71b** on the inside of the cover, in an analogous manner as with the first two rows **64a, 64b** in the indicium of Figure 8. This allows the monitor to determine the position of the cover. However, in this case, the positions and sizes of the markings correspond to particular opening angles of the cover.

The position of the cover during the first stage of opening is monitored by means of the optical sensors **22** on the inner side of the monitor which detect the motion of the indicia on the blister strip. Monitoring is handed over to the external optical sensor **24** for the second stage in which the blister strip does not move. The external optical sensor is preferably switched on shortly before the indexing mechanism is disengaged, at which point the cover has opened far enough to cover the external optical sensor. This saves battery power because the external optical sensor is only switched on when needed. It also prevents false readings, which could otherwise occur e.g. if the user puts their fingers over the external optical sensor.

Figure 10B shows the external sensor voltages resulting from the markings on the inside of the cover as function of its opening angle. Low voltages correspond to the cavities and high voltages to the other regions. Low and high voltages (rather than transitions) from the first sensor are coded as 1 and 2, and from the second sensor as 3 and 4 respectively. The external optical sensor is switched on when the cover has reached an angle of about 80°, at which point the blister strip is being indexed, close to the end of the first stage of opening. The code from the markings at this point is 23. If actuation continues normally, the code changes to 24 at about 90°, which is the end of the first stage of opening, i.e. the end of indexing and the start of piercing. When the cover has been fully opened (about 110°) at the end of the second stage, the code changes to 14 and then shortly afterwards to 13; this indicates that the blister has been pierced. However, If the user closes the cover part way through the first stage so that actuation is aborted before piercing, the code changes from 23 to 13 and then shortly afterwards to 14. Thus, the markings allow the monitor to determine the position of the cover using a similar method to that described above for the blister strip, even if the user moves the cover backwards and forwards before committing to piercing or aborting the actuation.

The monitor has a power source, such as a rechargeable battery. The monitor may have a motion sensor, such as an accelerometer, and means for switching on the monitor when motion is detected. The motion sensor may be configured to sense a specific gesture, such as picking up the monitor. Alternatively, a reed switch and a corresponding magnet on the mouthpiece cover, or a mechanical switch which interacts with the cover can be used to switch the monitor on. This avoids the need for the monitor to be permanently switched on, and hence conserves battery power.

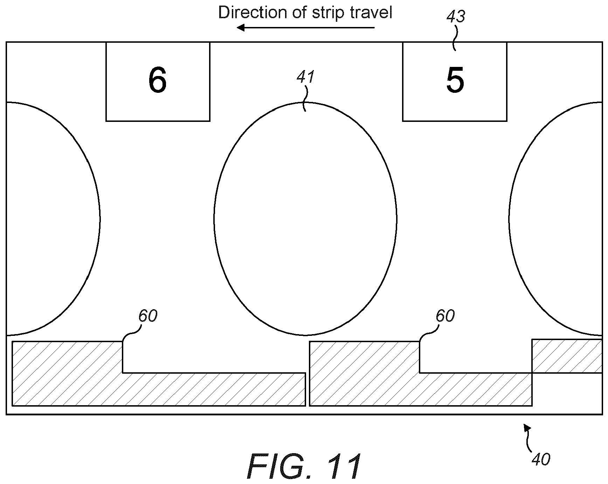

Figures 11 to 13 relate to a second embodiment in which the indicium is in the form of a 2D matrix code. Figure 11 shows part of a blister strip **40** with printed numbers **43** and 2D matrix code indicia **60.** Each indicium **60** encodes a unique number associated with each individual blister; the blister number **43** may also be printed on the blister strip, so that it can be read by the user through the opening **6** window in the housing on the opposite side of the inhaler from the monitor, as with the blister strip of Figure 3.

As with the previous embodiment, the sensors are held in a fixed position above the aperture **11** in the housing of the inhaler, corresponding to the location of the indicia **60** on the blister strip. When the user actuates the inhaler, the blister strip **40** is indexed from right to left so that the indicia move past the sensor. Instantaneous readings of parts of the 2D matrix code are taken at certain points during opening of the cover. The matrix code encodes the blister number, but (in contrast to the previous embodiment) it does not provide information on the position and direction of motion of the blister strip. Instead, these are determined by means of two mechanical switches on the monitor which are triggered by motion of the cover, as described below.

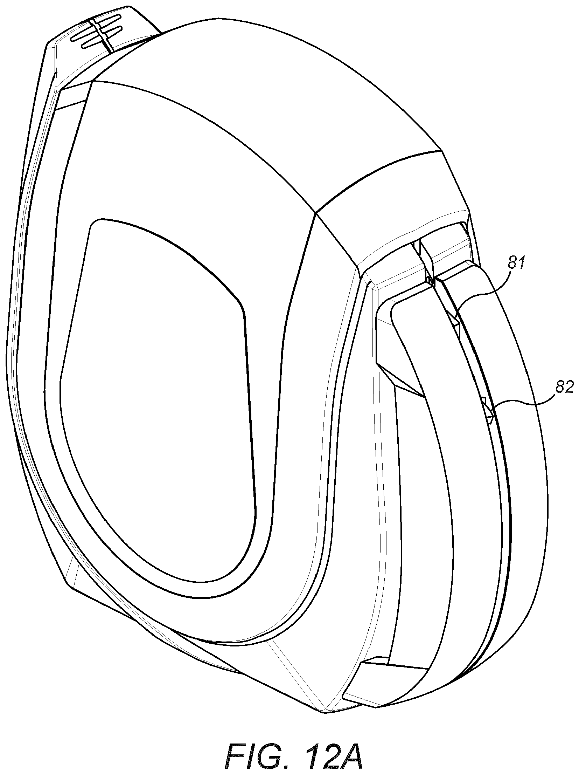

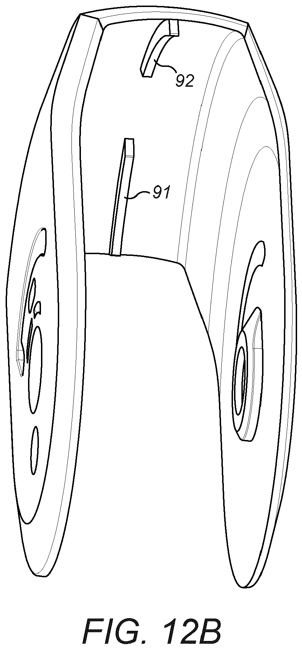

Figure 12A shows the inhaler and monitor and Figure 12B shows the cover removed from the inhaler, so that its inside is visible. The monitor has two mechanical switches **81, 82** (instead of the external optical sensor). The inside of the cover has two cams **91, 92** which come into and out of contact with the switches as the cover is opened, thereby changing the states of the switches. When one of the cams comes into contact with one of the switches, the switch state is changed from 0 to 1; then as the cam moves past the switch and ceases to be in contact with it, the state of the switch changes back from 1 to 0. The switches **81,82** and cams **91, 92** are arranged so that the states of the two switches are 90° out of phase with each other, i.e. they form a quadrature encoder. Since each cam causes two changes of state in each switch, there is a total of 2 x 2 x 2 = 8 changes of switch state, i.e. two complete cycles of quadrature logic. The monitor determines the position and direction of motion of the cover during opening and closing from the sequence of switch states.

The switches are positioned at opening angles of 17 and 32°. The length of the cams correspond to angles of 25° and 18°, and the angular gap between the end of the first cam and the start of the second cam is 26°. These are chosen to ensure that the first and second switches do not change state at the same opening angle. The sequence of switch states as the cover is opened is as shown in Table 3.

**Table 3**

| **Opening angle (°)** | 17 | 32 | | 42 | 57 | 68 | 83 | 86 | 101 |
|---|---|---|---|---|---|---|---|---|---|
| **Switch 1 contact** | | Cam 1 | | | | Cam 2 | | | |
| **Switch 1 state** | 0 | 1 | | 0 | | 1 | | 0 | |
| **Switch 2 contact** | | | Cam 1 | | | | Cam 2 | | |
| **Switch 2 state** | 0 | | 1 | | 0 | | 1 | | 0 |
| **Switch states** | 00 | 10 | 11 | 01 | 00 | 10 | 11 | 01 | 00 |
| **Position variable** | 0 | 1 | 2 | 3 | 4 | 5 | 6 | 7 | 8 |
| **Read event (3 reads)** | | | | | 1 | 2 | | 3 | |
| **Read event (2 reads)** | | | | | 1 | | | 2 | |

When the cover is in the closed position (0°), neither cam is in contact with the switches. When the opening angle reaches 17°, the first cam **91** comes into contact with the first switch **81,** causing the first switch to change state from 0 to 1. When the opening angle reaches 32°, the first cam **91** comes into contact with the second switch **82,** causing the second switch to change state from 0 to 1. The first cam **91** is in contact with both switches as the cover moves from 32° to 42° (because the length of the first cam corresponds to an angle of 25°). At 42°, the first cam **91** passes the first switch **81** and comes out of contact with it, so the first switch **81** changes state from 1 to 0. At 57°, the first cam **91** passes the second switch **82** and comes out of contact with it, so the second switch **82** changes state from 1 to 0. Then, at 68°, the second cam **92** comes into contact with the first switch **81** causing the first switch to change state from 0 to 1. When the opening angle reaches 83°, the second cam **92** comes into contact with the second switch **82,** causing the second switch to change state from 0 to 1. The second cam **92** is in contact with both switches **81, 82** as the cover moves from 83° to 86° (because the length of the second cam **92** corresponds to an angle of 18°). At 86°, the second cam **92** passes the first switch **81** and comes out of contact with it, so the first switch changes state from 1 to 0. This corresponds to the end of the first stage of opening of the cover during which the blister strip is indexed. At 101°, the second cam **92** passes the second switch **82** and comes out of contact with it, so the second switch changes state from 1 to 0. This sequence of eight switch state changes defines nine positions in the opening sequence. During the rest of the opening (i.e. up to about 110°), both switches remain in state 0.

The monitor is able to distinguish between opening and closing of the cover. In the opening motion the sequence of switch states is always 00 10 11 01 00 and in closing it is always 00 01 11 10 00. Thus, for example, if 00 is followed by 10, the monitor knows that the cover is opening, whereas if 00 is followed by 01 it must be closing. Similarly 01 followed by 00 is opening and followed by 11 is closing; 11 followed by 01 is opening and followed by 10 is closing; and 10 followed by 11 is opening and followed by 00 is closing.

The monitor records a position variable, which is initially set to zero (i.e. when the monitor is switched on for the first time). The monitor then increments the position variable each time that one of the switches changes state change as the cover is opened, and similarly decrements it each time that one of the switches changes state change as the cover is closed. The position variable thus indicates the opening angle (position variable 1 corresponds to an angle of between 17° and 32°, position variable 2 corresponds to 32° to 42° etc.), so that the monitor can track the position of the cover.

The monitor can be switched on, or woken up from a sleep state, whenever one of the switches changes state. This avoids the need for the monitor to be permanently switched on, and hence conserves battery power, but without requiring a separate type of sensor, such as an accelerometer for this purpose. For example, if the patient uses the inhaler, but forgets to close the cover after use, the monitor may be configured to enter a sleep state after a certain period of time for which in the inhaler is inactive. When the inhaler is next used, the monitor knows that the position variable is one position away in either the opening or the closing direction from the position variable when it went to sleep, because it was woken up by the first switch state change. It can compare the current switch states with the switch states one position either side of the last known position, and hence unambiguously determine the positon of the cover.

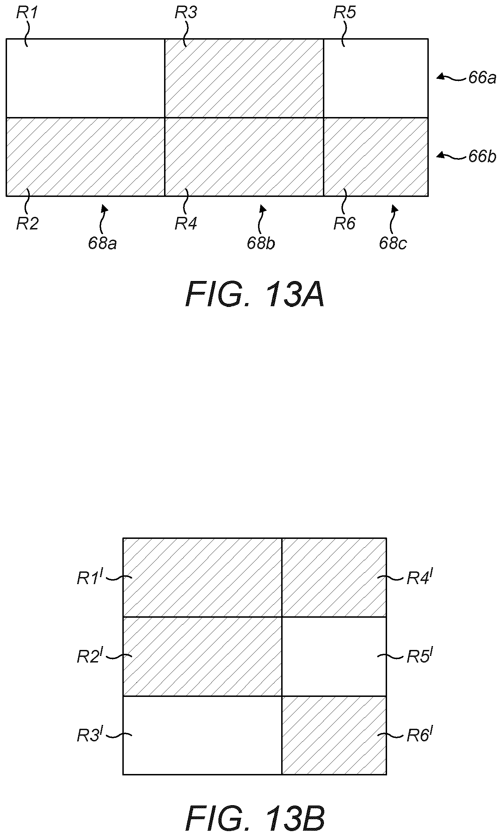

Figure 13A shows an indicium in the form of a 2D matrix code which defines an individual number associated with each blister in a 30 dose blister strip. The indicium consists of two rows **66a, 66b** and three columns **68a, 68b, 68c,** which define six regions (denoted R1 in row 66a, column 68a; R2 in row 66b, column 68a, R3 in row 66a, column 68b, R4 in row 66b, column 68b, R5 in row 66a, column 68c, and R6 in row 66b, column 68c). The regions are either printed black to form rectangular blocks, or left blank so that the reflective metal foil is exposed. The monitor correspondingly has two sensors, one for each row. There are three read events during the opening of the cover, one for each column. The read events preferably happen in the latter half of the first stage of opening, in order to allow time for the monitor to boot up after it wakes from a sleep state when the first switch is actuated on opening. Thus the read events may take place, for example, in positions 4, 5 and 7. In each read event, the change of switch state causes the monitor to turn the sensors **22** on for a short period of time (e.g. 1 ms). Since the optical sensors only need to be switched for a brief period during the read events, the power consumption is minimize and the battery life is extended.

The barcode is printed in the appropriate location on the blister strip so that each column is adjacent to the aperture in the housing when the cover is at the angle at which that column's read event occurs. Thus, the first column **68a** is adjacent to the aperture in position 4 (i.e. at 57°). At this point, the second switch changes state from 1 to 0, and the monitor turns on the sensors **22** to take an instantaneous reading of the first column. Similarly, the second column **68b** is adjacent to the aperture at position 5 (68°) and the third column **68c** at position 7 (86°).

In principle, since each row is read at a defined opening angle of the cover, i.e. a defined position along the blister strip, the columns need not be very wide. However, while the third read event may conveniently happen at or after the point at which the blister strip stops moving (i.e. the end of the first stage of actuation), the blister strip is in motion during at least the first and second read events. The width of the column in the direction of motion of the blister strip is therefore preferably greater than the distance that the blister strip travels in the time in which the sensors are on, to ensure that only the relevant column is read in each read event. In fact, the barcode may occupy the whole of the available length on the blister strip (i.e. the distance between adjacent blisters), with the centre of each row corresponding to the point at which it is expected to be read. Consequently, there is no little or blank space between the indicia of adjacent blisters, as is apparent in Figure 11. Maximising the width of each column allows the code to be read while the blister strip is moving and also makes the reading robust to small variations that may occur in the printing position on the blister strip, the blister strip position within the inhaler and / or the position of the sensors on the monitor.

The first region (R1) in the first column **68a** is a parity block, and the other region (R2) encodes the first digit of a five digit binary number. The regions (R3 & R4, R5 & S6) in the second **68b** and third **68c** columns respectively encode the other four digits. The parity block is chosen so that there is always an even number of printed and blank blocks, and so can be used for error checking. The number of printed blocks in R2 to R6 is counted; if this number is even, the parity block should be blank, and if the number is odd, the parity block should be printed. Once the encoded blister number has been read, the expected parity block value can be compared with the actual parity block value. If they disagree, the monitor determines that an error has occurred. In that case, instead of reading the binary number from the blister strip, the monitor can derive the expected blister number from the previously read blister number.

In Figure 13A, in the first column **68a** R1 is blank and R2 is printed; in the second column **68b** R3 and R4 are printed; and in the third column **68c,** R5 is blank and R6 is printed. Table 4 summarizes the resulting output voltage signals (low or high) from the two sensors. The output voltage is proportional to the intensity of the light reflected from the indicium that is received by each sensor. The binary state is determined by comparing the output voltage to a threshold level. If the output voltage is higher than the threshold voltage then the state is 1 and if the voltage is lower the state is 0. The outputs resulting from the indicium of Figure 13A when the blister strip is indexed through a complete actuation are shown in Table 4 below. The indicium encodes the binary number 00010, i.e. 2. The parity block in S1 is 1, which confirms that there is an even number (4) of printed blocks, i.e. zeros in the binary number.

**Table 4**

| **Region** | R1 | R2 | R3 | R4 | R5 | R6 |
|---|---|---|---|---|---|---|
| **Blocks** | | | | | | |
| **Sensor voltage** | High | Low | Low | Low | High | Low |
| **Binary number** | 1 | 0 | 0 | 0 | 1 | 0 |

Figure 13B shows an alternative form of a 2D matrix code which consists of three rows and two columns. The monitor correspondingly has three sensors, one for each row and there are two read events, one read event for each column, for example at positions 4 and 7.

The first region (R1') is a parity block, and the other blocks (R2' to R6') encode a five digit binary number. In the first column, R1' and R2' are printed while R3' is blank; in the second column, R4' and R6' are printed, and R5' is blank. The resulting sensor outputs are shown in Table 5. The indicium encodes the binary number 01010, i.e. 10. The parity block in S1 is 0, which confirms that there is an odd number (3) of printed blocks, i.e. zeros in the binary number.

**Table 5**

| **Region** | R1' | R2' | R3' | R4' | R5' | R6' |
|---|---|---|---|---|---|---|
| **Blocks** | | | | | | |
| **Sensor voltage** | Low | Low | High | Low | High | Low |
| **Binary number** | 0 | 0 | 1 | 0 | 1 | 0 |

The indicia in Figures 13A and 13B can alternatively be used to define individual numbers associated with each blister in a 60 dose blister strip by using the parity block to represent a sixth digit in the binary number.

As with the previous embodiment, the indicia could have grey printed regions in addition to black and unprinted regions to represent three values (0, 1 and 2). This has the advantage that a single region can contain more information, so fewer regions are required to encode the blister number (only four regions are needed for numbers up to 81, including 30 and 60, which can be read with two optical sensors in the monitor and two read events). On the other hand, using only black printing has the advantage of being more robust to any variability in the reflected light from the blister strip, e.g. due to variations in the quality of the print or the position of the blister strip within the inhaler, etc..

An incomplete actuation could result in only part of the indicium being read, for example only the first row. If the actuation is then aborted and the cover closed, the monitor is able to recognise the reverse motion of the blister strip (as described above), and hence it can delete the digits in the binary number that had been read, in order to prevent an incorrect reading from being recorded. Thus if the user were to reverse the direction of motion of the cover part way through actuation, but then go on to complete the full actuation, the binary number would be partially read, then deleted, and then read correctly, even though actuation is aborted or reversed part way through.

The monitor may (in either embodiment) also have a pressure sensor 26, which is located in a recess on the inside face (see Figure 2B). The pressure sensor abuts the orifice **12** in the housing (see Figure 1D), which leads via a channel in the inhaler housing to the mouthpiece. Alternatively, the pressure sensor could be connected to the mouthpiece via a separate external tube. The monitor can thereby measure the pressure in the mouthpiece to sense the user's inhalation.

The monitor has a controller and memory (e.g. a suitable microprocessor) which are configured to process and/or store information read from the sensors and / or switches relating to patient's usage of the inhaler. The monitor may also include means for transmitting information from the sensors and / or switches to an external device, such as a computer or smartphone, e.g. via Bluetooth^{®}. The information may then be displayed to the user and / or a medical professional, by means of suitable software, for example a smartphone app. The information may additionally or alternatively be stored on the monitor for subsequent interrogation, and / or transmitted to an online health platform. The monitor may also include means for receiving information from an external device.

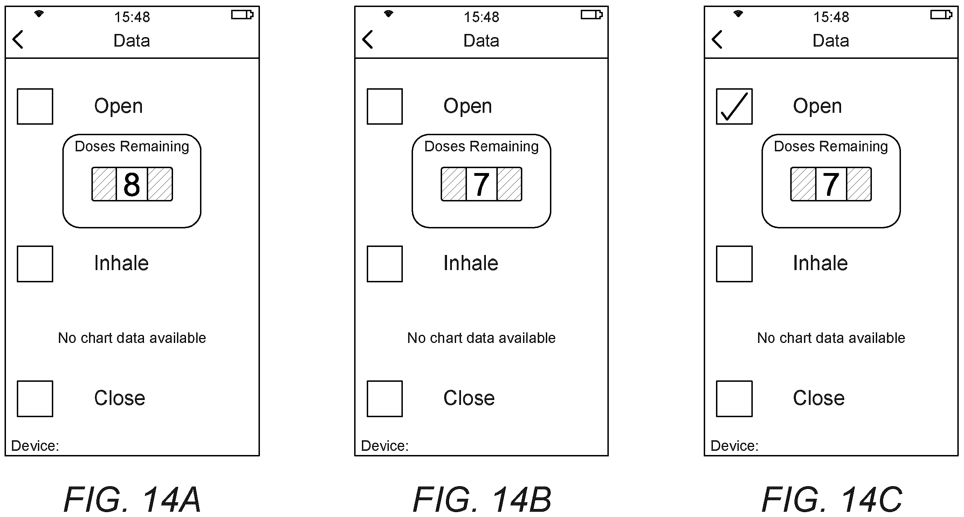

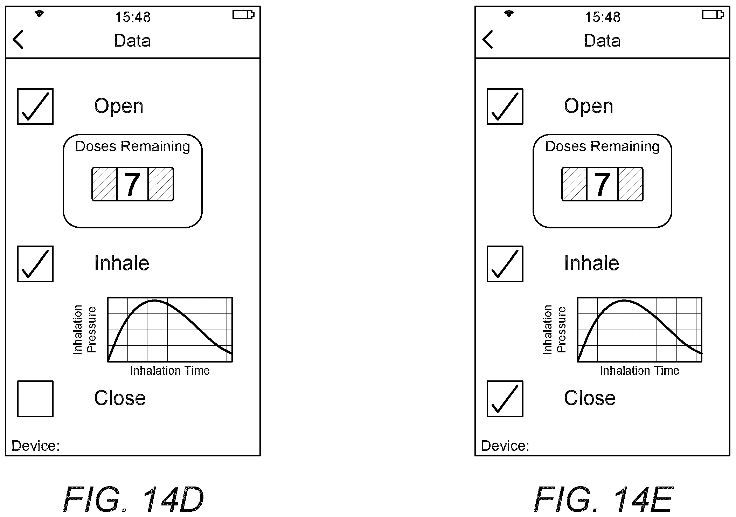

Figure 14 shows a series of displays on a mobile phone with an app that receives data from the monitor as the patient uses the inhaler. Figure 14A shows the screen before the inhaler is actuated. The number of doses remaining is displayed. Figure 14B shows the display at the end of the first stage of opening the cover. The dose counter has been decreased by one. If actuation is aborted at this stage (i.e. before piercing) by closing the cover, the dose counter is increased by one back to the previous value. Figure 14C shows the display when the cover has been fully opened (i.e. at the end of the second stage of opening) so that piercing has taken place. This is indicated by a tick in the "Open" box, as a result of a signal from the external optical sensor **24** in the first embodiment or from the switches **81, 82** in the second embodiment. Figure 14D shows the display after the user has inhaled, indicated by a tick in the "Inhale" box and the appearance of a chart showing the inhalation pressure recorded by the pressure sensor **26** as a function of time. If no inhalation is detected after a pre-defined period of time, or if the cover is closed before inhalation is detected, a cross would appear in the "Inhale" box. Figure 14E shows the display after the cover has been closed, indicated by a tick in the "Close" box, also a result of a signal from the external optical sensor **24** in the first embodiment or from the switches **81, 82** in the second embodiment. If the cover is not closed within a pre-defined time, a cross would appear in the "Close" box.

The monitor and / or app may also be configured to convey instructions to the user, such as an audible or visible reminder message to obtain a new inhaler if there are, for example, fewer than five doses remaining. The app could also provide instructions to the user concerning how to inhale correctly.

While the particular inhaler described above uses a blister strip, the invention can equally be used for inhalers which use different types of blister pack, such as a blister disk. The principle of absolute blister counting by associating a unique, machine-readable indicium on the pack with each dose (e.g. each blister or pair of blisters) so that the number of doses that have been dispensed,or remain to be dispensed, can be determined, even if the monitor is removed from the inhaler and later reattached, applies equally to these.

The medicament is suitable for administration by inhalation, for example for the treatment of a respiratory disease. It may include one of more of the following classes of pharmaceutically active material: anticholinergics, adenosine A2A receptor agonists, β2-agonists, calcium blockers, IL-13 inhibitors, phosphodiesterase-4-inhibitors, kinase inhibitors, steroids, CXCR2, proteins, peptides, immunoglobulins such as Anti-IG-E, nucleic acids in particular DNA and RNA, monoclonal antibodies, small molecule inhibitors and leukotriene B4 antagonists. The medicament include excipients, such as fine excipients and / or carrier particles (for example lactose), and / or additives (such as magnesium stearate, phospholipid or leucine).

Suitable β2-agonists include albuterol (salbutamol), preferably albuterol sulfate; carmoterol, preferably carmoterol hydrochloride; fenoterol; formoterol; milveterol, preferably milveterol hydrochloride; metaproterenol, preferably metaproterenol sulfate; olodaterol; procaterol; salmeterol, preferably salmeterol xinafoate; carmoterol; terbutaline, preferably terbutaline sulphate; vilanterol, preferably vilanterol trifenatate or indacaterol, preferably indacaterol maleate.

Suitable steroids include budesonide; beclamethasone, preferably beclomethasone dipropionate; ciclesonide; fluticasone, preferably fluticasone furoate; mometasone, preferably mometasone furoate. In one aspect, the method comprises jet milling mometasone, preferably mometasone furoate in the presence of a liquid aerosol.

Suitable anticholinergics include: aclidinium, preferably aclidinium bromide; glycopyrronium, preferably glycopyrronium bromide; ipratropium, preferably ipratropium bromide; oxitropium, preferably oxitropium bromide; tiotropium, preferably tiotropium bromide; umeclidinium, preferably umeclidinium bromide; Darotropium bromide; or tarafenacin.

The active material may include double or triple combinations such as salmeterol xinafoate and fluticasone propionate; budesonide and formoterol fumarate dihydrate glycopyrrolate and indacaterol maleate; glycopyrrolate, indacaterol maleate and mometasone furoate; fluticasone furoate and vilanterol; vilanterol and umclidinium bromide; fluticasone furoate, vilanterol and umclidinium bromide.

## Claims

1. A dry powder inhaler (1) and a monitor (20), the dry powder inhaler having a mouthpiece (5) and a housing (2,3) which contains a blister pack (40), comprising a plurality of blisters (41), wherein a blister, or group of blisters, provides a dose of powdered medicament for inhalation, wherein the blister pack has non-numerical indicia (42, 46, 54, 60) which encode an individual, unique number that is associated with each dose, wherein the inhaler is adapted for removably mounting the monitor, wherein the monitor comprises one or more sensors (22) which are configured to read the non-numerical indicia, and a controller and memory which are configured to process and/or store information read by the sensors so that the monitor can determine the unique number of each dose.

2. A dry powder inhaler and a monitor according to claim 1 wherein a dose of medicament is provided by single blisters and wherein a non-numerical indicium is associated with each blister.

3. A dry powder inhaler and a monitor according to claim 1 wherein a dose of medicament is provided by pairs of blisters and wherein a non-numerical indicium is associated with each pair.

4. A dry powder inhaler and a monitor according to any of claims 1 to 3 wherein the indicia comprise three rows of printed blocks.

5. A dry powder inhaler and a monitor according to claim 4 wherein the indicia comprise a 2D matrix code (54, 60).

6. A dry powder inhaler and a monitor according to any of claims 1 to 5, wherein the blister pack is a blister strip.

7. A dry powder inhaler and a monitor according to claim 6 wherein the inhaler further comprises a mouthpiece cover (4), wherein:
• in a first stage, moving the mouthpiece cover from the closed position to an intermediate position causes the blister strip to be advanced;
• in a second stage, moving the mouthpiece cover from the intermediate position to the open position causes a piercer to pierce one or more blisters.

8. A dry powder inhaler and a monitor according to claim 7 wherein the inside of the mouthpiece cover (4) has one or more cams (91,92) for actuating one or more switches (81,82) on the monitor as the cover is moved for determining the position and / or direction of motion of the cover.

9. A monitor (20) which is removably attachable to an inhaler (1) according to any of claims 1 to 8, wherein the monitor comprises one or more sensors (22) which are configured to read non-numerical indicia (42, 46, 54, 60) which encode an individual, unique number that is associated with each dose in a blister pack (40) in the inhaler, and a controller and memory which are configured to process and/or store information read by the sensors so that the monitor can determine the unique number of each dose.

10. A monitor according to claim 9 which is configured to read the indicia as the blister pack is indexed.

11. A monitor according to claim 10 which has three optical sensors (22) for reading indicia when the monitor is mounted on an inhaler according to any of claims 4 to 8.

12. A monitor according to any of claims 9 to 11 which is attachable to an inhaler according to claim 8, wherein the monitor has one or more switches (81,82) on its outer side which are configured to be actuated by the cam(s) (91,92) on the inside of the mouthpiece cover (4) of the inhaler.

13. A monitor according to any of claims 9 to 12 which has a pressure sensor (26) for detecting inhalation on the mouthpiece when the monitor is mounted on an inhaler according to any of claims 1 to 8.

14. A dry powder inhaler (1) and a monitor (20) according to any of claims 1 to 8, wherein the monitor is removably attached to the inhaler.

## Patentansprüche

1. Trockenpulverinhalator (1) und Überwachungseinrichtung (20), wobei der Trockenpulverinhalator ein Mundstück (5) und ein Gehäuse (2, 3) aufweist, das eine Blisterpackung (40) enthält, die eine Mehrzahl von Blistern (41) umfasst, wobei ein Blister oder eine Gruppe von Blistern eine Dosis eines pulverförmigen Arzneimittels zur Inhalation bereitstellt, wobei die Blisterpackung nicht-numerische Zeichen (42, 46, 54, 60) aufweist, die eine individuelle eindeutige Nummer codieren, die mit jeder Dosis assoziiert ist, wobei der Inhalator zur abnehmbaren Befestigung der Überwachungseinrichtung geeignet ist, wobei die Überwachungseinrichtung einen oder mehrere Sensoren (22), die zum Lesen der nicht-numerischen Zeichen ausgelegt sind, und eine Steuerung und einen Speicher umfasst, die zum Verarbeiten und/oder Speichern von Informationen ausgelegt sind, die von den Sensoren gelesen werden, sodass die Überwachungseinrichtung die eindeutige Nummer jeder Dosis bestimmen kann.

2. Trockenpulverinhalator und Überwachungseinrichtung nach Anspruch 1, wobei eine Arzneimitteldosis durch einzelne Blister bereitgestellt wird und wobei ein nichtnumerisches Zeichen mit jedem Blister assoziiert ist.

3. Trockenpulverinhalator und Überwachungseinrichtung nach Anspruch 1, wobei eine Arzneimitteldosis durch Blisterpaare bereitgestellt wird und wobei ein nichtnumerisches Zeichen mit jedem Paar assoziiert ist.

4. Trockenpulverinhalator und Überwachungseinrichtung nach einem der Ansprüche 1 bis 3, wobei die Zeichen drei Reihen gedruckter Blöcke umfassen.

5. Trockenpulverinhalator und Überwachungseinrichtung nach Anspruch 4, wobei die Zeichen einen 2D-Matrixcode (54, 60) umfassen.

6. Trockenpulverinhalator und Überwachungseinrichtung nach einem der Ansprüche 1 bis 5, wobei die Blisterpackung ein Blisterstreifen ist.

7. Trockenpulverinhalator und Überwachungseinrichtung nach Anspruch 6, wobei der Inhalator ferner eine Mundstückabdeckung (4) umfasst, wobei:
• in einer ersten Phase das Bewegen der Mundstückabdeckung von der geschlossenen Position in eine Zwischenposition ein Vorschieben des Blisterstreifens bewirkt;
• in einer zweiten Phase das Bewegen der Mundstückabdeckung von der Zwischenposition in die offene Position einen Locher zum Durchstechen eines oder mehrerer Blister veranlasst.

8. Trockenpulverinhalator und Überwachungseinrichtung nach Anspruch 7, wobei die Innenseite der Mundstückabdeckung (4) eine oder mehrere Nocken (91, 92) zum Betätigen eines oder mehrerer Schalter (81, 82) auf der Überwachungseinrichtung aufweist, wenn die Abdeckung bewegt wird, um die Position und/oder die Bewegungsrichtung der Abdeckung zu bestimmen.

9. Überwachungseinrichtung (20), die abnehmbar an einem Inhalator (1) nach einem der Ansprüche 1 bis 8 angebracht werden kann, wobei die Überwachungseinrichtung einen oder mehrere Sensoren (22), die zum Lesen von nicht-numerischen Zeichen (42, 46, 54, 60) ausgelegt sind, die eine individuelle eindeutige Nummer codieren, die mit jeder Dosis in einer Blisterpackung (40) in dem Inhalator assoziiert ist, und eine Steuerung und einen Speicher umfasst, die zum Verarbeiten und/oder Speichern von Informationen ausgelegt sind, die von den Sensoren gelesen werden, sodass die Überwachungseinrichtung die eindeutige Nummer jeder Dosis bestimmen kann.

10. Überwachungseinrichtung nach Anspruch 9, die zum Lesen der Zeichen ausgelegt ist, wenn die Blisterpackung indexiert wird.

11. Überwachungseinrichtung nach Anspruch 10, die drei optische Sensoren (22) zum Lesen von Zeichen aufweist, wenn die Überwachungseinrichtung an einem Inhalator nach einem der Ansprüche 4 bis 8 befestigt ist.

12. Überwachungseinrichtung nach einem der Ansprüche 9 bis 11, die an einem Inhalator nach Anspruch 8 angebracht werden kann, wobei die Überwachungseinrichtung einen oder mehrere Schalter (81, 82) auf ihrer Außenseite aufweist, die dazu ausgelegt sind, durch den oder die Nocken (91, 92) auf der Innenseite der Mundstückabdeckung (4) des Inhalators betätigt zu werden.

13. Überwachungseinrichtung nach einem der Ansprüche 9 bis 12, die einen Drucksensor (26) zum Erfassen von Inhalation an dem Mundstück aufweist, wenn die Überwachungseinrichtung an einem Inhalator nach einem der Ansprüche 1 bis 8 befestigt ist.

14. Trockenpulverinhalator (1) und Überwachungseinrichtung (20) nach einem der Ansprüche 1 bis 8, wobei die Überwachungseinrichtung abnehmbar am Inhalator angebracht ist.

## Revendications

1. Inhalateur de poudre sèche (1) et dispositif de surveillance (20), l'inhalateur de poudre sèche ayant un embout buccal (5) et un boîtier (2, 3) qui contient un emballage-coque (40), comprenant une pluralité de coques (41), une coque ou un groupe de coques fournissant une dose de médicament en poudre à inhaler, l'emballage-coque ayant des symboles non numériques (42, 46, 54, 60) qui encodent un numéro individuel, unique qui est associé à chaque dose, l'inhalateur étant adapté pour monter de façon amovible le dispositif de surveillance, le dispositif de surveillance comprenant un ou plusieurs capteurs (22) qui sont configurés pour lire les symboles non numériques, et un contrôleur et une mémoire qui sont configurés pour traiter et/ou stocker des informations lues par les capteurs de telle sorte que le dispositif de surveillance peut déterminer le numéro unique de chaque dose.

2. Inhalateur de poudre sèche et dispositif de surveillance selon la revendication 1 dans lesquels une dose de médicament est fournie par des coques individuelles et dans lesquels un symbole non numérique est associé à chaque coque.

3. Inhalateur de poudre sèche et dispositif de surveillance selon la revendication 1 dans lesquels une dose de médicament est fournie par des paires de coques et dans lesquels un symbole non numérique est associé à chaque paire.

4. Inhalateur de poudre sèche et dispositif de surveillance selon l'une quelconque des revendications 1 à 3 dans lesquels les symboles comprennent trois rangées de blocs imprimés.

5. Inhalateur de poudre sèche et dispositif de surveillance selon la revendication 4 dans lesquels les symboles comprennent un code matriciel 2D (54, 60).

6. Inhalateur de poudre sèche et dispositif de surveillance selon l'une quelconque des revendications 1 à 5, dans lesquels l'emballage-coque est une bande de coques.

7. Inhalateur de poudre sèche et dispositif de surveillance selon la revendication 6, l'inhalateur comprenant en outre un couvercle d'embout buccal (4), dans lesquels :
• dans une première phase, le déplacement du couvercle d'embout buccal de la position fermée à une position intermédiaire fait avancer la bande de coques ;
• dans une deuxième phase, le déplacement du couvercle d'embout buccal de la position intermédiaire à la position ouverte conduit un poinçon à percer une ou plusieurs coques.

8. Inhalateur de poudre sèche et dispositif de surveillance selon la revendication 7 dans lesquels l'intérieur du couvercle d'embout buccal (4) a une ou plusieurs cames (91, 92) destinées à actionner un ou plusieurs commutateurs (81, 82) sur le dispositif de surveillance lorsque le couvercle est déplacé pour déterminer la position et/ou la direction de déplacement du couvercle.

9. Dispositif de surveillance (20) qui peut être attaché de façon amovible à un inhalateur (1) selon l'une quelconque des revendications 1 à 8, le dispositif de surveillance comprenant un ou plusieurs capteurs (22) qui sont configurés pour lire des symboles non numériques (42, 46, 54, 60) qui encodent un numéro individuel, unique qui est associé à chaque dose dans un emballage-coque (40) dans l'inhalateur, et un contrôleur et une mémoire qui sont configurés pour traiter et/ou stocker des informations lues par les capteurs de telle sorte que le dispositif de surveillance peut déterminer le numéro unique de chaque dose.

10. Dispositif de surveillance selon la revendication 9 qui est configuré pour lire les symboles lorsque l'emballage-coque est indexé.

11. Dispositif de surveillance selon la revendication 10 qui a trois capteurs optiques (22) destinés à lire des symboles quand le dispositif de surveillance est monté sur un inhalateur selon l'une quelconque des revendications 4 à 8.

12. Dispositif de surveillance selon l'une quelconque des revendications 9 à 11 qui peut être attaché à un inhalateur selon la revendication 8, le dispositif de surveillance ayant un ou plusieurs commutateurs (81, 82) sur son côté extérieur qui sont configurés pour être actionnés par la/les came(s) (91, 92) sur l'intérieur du couvercle d'embout buccal (4) de l'inhalateur.

13. Dispositif de surveillance selon l'une quelconque des revendications 9 à 12 qui a un capteur de pression (26) destiné à détecter une inhalation sur l'embout buccal quand le dispositif de surveillance est monté sur un inhalateur selon l'une quelconque des revendications 1 à 8.

14. Inhalateur de poudre sèche (1) et dispositif de surveillance (20) selon l'une quelconque des revendications 1 à 8, le dispositif de surveillance étant attaché de façon amovible à l'inhalateur.
